# EUROPÄISCHE PATENTANMELDUNG

(11) **EP 0 589 382 A1**
(43) Veröffentlichungstag der Anmeldung: **30.03.1994**
(21) Anmeldenummer: 93115078.3
(22) Anmeldetag: 20.09.1993
(51) Int. Cl.: C07C 231/12, C07C 227/08

(54) **Verfahren zur Herstellung von reinen wässrigen Betainlösungen**

(30) Priorität: 25.09.1992 DE 4232157
(71) Anmelder: HOECHST AKTIENGESELLSCHAFT, D-65926 Frankfurt am Main (DE)
(72) Erfinder: Seitz, Hubert, Dr., D-84508 Burgkirchen (DE); Vybiral, Reinhard, Dr., D-84508 Burgkirchen (DE)

(57) **Zusammenfassung**

Das beschriebene Verfahren betrifft die Herstellung von wäßrigen Betainlösungen durch Umsetzung von einem tertiären Amin mit einer ω-Monohalogencarbonsäure, vorzugsweise Monochloressigsäure, und Alkalimetallhydroxid in Gegenwart von Wasser, die insbesondere bezüglich tertiärem Ausgangsamin und ω-Monohalogencarbonsäure hochrein sein sollen. Dies wird erreicht, indem die drei Reaktionskomponenten zunächst in einem bestimmten Molverhältnis umgesetzt werden, mit dem Ziel, eine Betainlösung zu erhalten, die nur noch eine tolerierte Menge an Ausgangsamin enthält. Diese wäßrige Betainlösung wird dann mit einem Sulfonierungsagens behandelt, vorzugsweise mit einem Alkalimetallsulfit, Alkalimetallpyrosulfit oder Alkalimetallbisulfit zur Umwandlung der vorhandenen ω-Monohalogencarbonsäure in die entsprechende, nicht störende Sulfocarbonsäure. Das beschriebene Verfahren ist einfach in der Durchführung und führt zu den erwähnten hochreinen wäßrigen Betainlösungen.

## Beschreibung

Die Erfindung betrifft ein Verfahren zur Herstellung von reinen wäßrigen Lösungen von Betainen der allgemeinen Formel 1 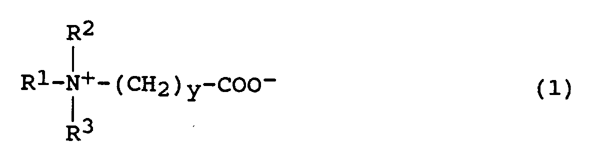
worin bedeuten
- R¹: einen Alkylrest mit 6 bis 22 C-Atomen, vorzugsweise 8 bis 18 C-Atomen, oder einen Rest der Formel R'CONH(CH₂)_{z}-, worin R' ein Alkylrest mit 5 bis 21 C-Atomen ist, vorzugsweise mit 5 bis 17 C-Atomen, und z 2, 3 oder 4 ist,
- R²: einen Alkylrest mit 1 bis 4 C-Atomen oder einen Rest der Formel -(CH₂)ₘ-OH, worin m 1, 2 oder 3 ist,
- R³: einen Alkylrest mit 1 bis 4 C-Atomen oder einen Rest der genannten Formel -(CH₂)ₘ-OH und
- y: 1, 2 oder 3,
durch Umsetzung von einem tertiären Amin der allgemeinen Formel 2

R¹-NR²R³ (2)

worin R¹, R² und R³ die angegebenen Bedeutungen haben, mit einer ω-Halogencarbonsäure der allgemeinen Formel 3

X-(CH₂)_{y}-COOH (3)

worin X ein Halogen ist, vorzugsweise Cl, und y die genannte Bedeutung hat,
und mit Alkalimetallhydroxid in wäßriger Phase.

Die Herstellung von wäßrigen Lösungen von Betainen durch Umsetzung (Quaternisierung) von tertiären Aminen mit einer ω-Halogencarbonsäure und Alkalimetallhydroxid in wäßriger Phase ist schon seit langem bekannt, zum Beispiel aus den beiden US-Patentschriften 3,819,539 und 4,497,825. Sie beruht auf der folgenden Gesamtreaktionsgleichung (die Reaktionskomponenten sind Dimethyllaurylamin, Monochloressigsäure und Natriumhydroxid): 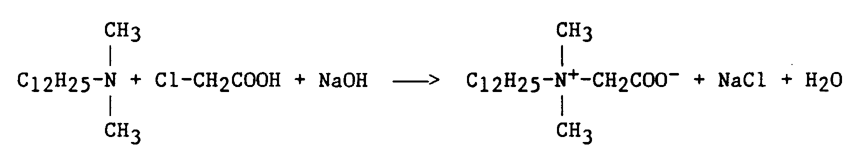
Die erhaltenen wäßrigen Lösungen bestehen im wesentlichen aus dem angestrebten Betain, dem gebildeten Alkalimetallhalogensalz und dem eingesetzten und gebildeten Wasser und haben im allgemeinen einen Wirkstoffgehalt von 20 bis 60 Gew.-%, vorzugsweise 25 bis 50 Gew.-%. Diese wäßrigen Betainlösungen stellen bereits als solche wertvolle Produkte dar (Waschrohstoffe), insbesondere für das Gebiet der Körperpflege. Betaine weisen nämlich nicht nur gute Reinigungseigenschaften, sondern auch gute Hautverträglichkeit auf.

Bei der Herstellung der in Rede stehenden wäßrigen Betainlösungen geht es vor allem darum, das Betain in hoher Ausbeute und hoher Reinheit zu erhalten. Die wäßrigen Betainlösungen sollen insbesondere bezüglich Ausgangsamin und Halogencarbonsäure (die als solche und/oder als Alkalimetallsalz vorliegt) rein sein, das heißt, sie sollen diese Verbindungen, wenn überhaupt, nur noch in einer sehr geringen Menge enthalten. Es ist schon häufig versucht worden, dieses Ziel durch spezielle Maßnahmen zu erreichen, so zum Beispiel durch Einhalten eines bestimmten pH-Wertes während der Quaternisierung, durch Zusammenbringen der Reaktionskomponenten tertiäres Amin, Halogencarbonsäure und Alkalimetallhydroxid in einer ganz bestimmten Reihenfolge, indem zum Beispiel das tertiäre Amin und die Halogencarbonsäure vorgelegt und das Alkalimetallhydroxid langsam zudosiert werden oder indem die Halogencarbonsäure und das Alkalimetallhydroxid vorgelegt und das tertiäre Amin zudosiert wird, ferner durch Verwendung spezieller Lösungsmittel und/oder durch Aufrechterhalten einer relativ niedrigen Temperatur während der Umsetzung. All diese Versuche brachten nicht den angestrebten Erfolg. Die mit den bekannten Verfahren erhaltenen wäßrigen Betainlösungen entsprechen nicht den erwähnten Reinheitsforderungen, die in neuerer Zeit immer strenger werden.

Die Aufgabe der Erfindung besteht demnach darin, ein Verfahren zur Herstellung der eingangs genannten wäßrigen Betainlösungen zur Verfügung zu stellen, das die Betaine in hoher Ausbeute und hoher Reinheit liefert, das heißt wäßrige Betainlösungen mit weniger als 1,5 Gew.-% tertiäres Amin, vorzugsweise weniger als 0,5 Gew.-%, und mit weniger als 50 ppm ω-Monohalogencarbonsäure, vorzugsweise weniger als 10 ppm.

Das erfindungsgemäße Verfahren ist dadurch gekennzeichnet, daß man zunächst das tertiäre Amin, die ω-Monohalogencarbonsäure und das Alkalimetallhydroxid im Molverhältnis von 1 zu 1 bis 1,5 zu 1 bis 1,8, vorzugsweise 1 zu 1,03 bis 1,3 zu 1 bis 1,5, bei einer Temperatur von 60 bis 98 °C, vorzugsweise 70 bis 95 °C, umsetzt und dann die erhaltene wäßrige Betainlösung bei einem pH-Wert von 7,5 bis 13, vorzugsweise 8 bis 10, und einer Temperatur von 60 bis 98 °C, vorzugsweise 70 bis 95 °C, mit einem Sulfonierungsagens behandelt zur Überführung der in der wäßrigen Betainlösung vorhandenen ω-Monohalogencarbonsäure in die ω-Sulfocarbonsäure.

Das erfindungsgemäße Verfahren beruht also auf der Kombination von zwei speziellen Verfahrensschritten. Im ersten Verfahrensschritt werden die Ausgangskomponenten, tertiäres Amin, ω-Monohalogencarbonsäure und Alkalimetallhydroxid, in einem ausgewählten Molverhältnis eingesetzt und die Umsetzung wird im allgemeinen bis zum genannten niedrigen Gehalt an Ausgangsamin geführt, das heißt, bis in der erhaltenen wäßrigen Betainlösung weniger als 1,5 Gew.-% tertiäres Amin, vorzugsweise weniger als 0,5 Gew.-% tertiäres Amin, bezogen auf eingesetztes tertiäres Amin, vorliegen. Mit dem erfindungsgemäßen Einsatz von äquimolarer oder überschüssiger Halogencarbonsäure, bezogen auf tertiäres Amin, wird also ein Reaktionsprodukt erreicht, das - wenn überhaupt - nur noch sehr wenig tertiäres Ausgangsamin enthält. Die im ersten Reaktionsschritt erhaltene wäßrige Betainlösung ist also rein bezüglich tertiäres Amin. Sie enthält aber Halogencarbonsäure, die als solche und insbesondere in Form eines Alkalimetallsalzes vorliegt (der Einfachheit halber wird im folgenden von Säure gesprochen). Um die wäßrige Betainlösung auch von Halogencarbonsäure frei zu machen, wird sie erfindungsgemäß mit einem Sulfonierungsmittel behandelt. Dabei wird die ω-Monohalogencarbonsäure in die entsprechende Sulfocarbonsäure übergeführt. Die nachstehende Reaktionsgleichung soll dies veranschaulichen, wobei die Halogencarbonsäure, die Monochloressigsäure und das Sulfonierungsagens Natriumhydrogensulfit ist (es versteht sich von selbst, daß die genannten Säuren als Alkalimetallsalze vorliegen):

Cl-CH₂COOH + NaHSO₃ → HSO₃-CH₂COOH + NaCl

Im Gegensatz zur ω-Monohalogencarbonsäure (zum Beispiel Monochloressigsäure) stellt die Sulfocarbonsäure (Sulfoessigsäure) keine störende Verbindung in der wäßrigen Betainlösung dar, sie ist im Gegensatz zur ω-Monohalogencarbonsäure insbesondere nicht toxisch. Nach der Sulfonierung liegt eine wäßrige Betainlösung vor, die sowohl bezüglich tertiäres Amin als auch bezüglich Halogencarbonsäure die angestrebte Reinheit aufweist. Sie besteht im wesentlichen aus dem gebildeten Betain, Alkalimetallhalogenid und Wasser und einer mehr oder weniger geringen Menge an Sulfocarbonsäure in Form eines Alkalimetallsalzes, wobei der Betaingehalt (Wirkstoffgehalt) etwa 20 bis 60 Gew.-% beträgt, vorzugsweise etwa 25 bis 50 Gew.-%.

Beim erfindungsgemäßen Verfahren wird also zunächst die Umsetzung zwischen dem tertiären Amin und der ω-Monohalogencarbonsäure in Gegenwart von Alkalimetallhydroxid und Wasser durchgeführt. Das tertiäre Amin, die ω-Monohalogencarbonsäure (sie wird im allgemeinen in Form einer 60 bis 80gew.%igen wäßrigen Lösung eingesetzt) und das Alkalimetallhydroxid (es wird im allgemeinen in Form einer 30 bis 60gew.%igen wäßrigen Lösung eingesetzt, vorzugsweise 35 bis 50gew.%igen wäßrigen Lösung) werden in einem Molverhältnis von 1 zu 1,0 bis 1,5 zu 1,0 bis 1,8 eingesetzt, vorzugsweise in einem Molverhältnis von 1 zu 1,03 bis 1,3 zu 1,0 bis 1,5. Die Menge an Wasser (das als solches und in Form der genannten wäßrigen Lösungen von Alkalimetallhydroxid und Halogencarbonsäure eingebracht wird) wird im allgemeinen so gewählt, daß die nach der Umsetzung erhaltene wäßrige Betainlösung den oben angegebenen Wirkstoffgehalt hat. Die Umsetzungstemperatur beträgt 60 bis 98 °C, vorzugsweise 70 bis 95 °C. Die Umsetzung wird im allgemeinen so lange aufrechterhalten, bis in der gebildeten wäßrigen Betainlösung kein tertiäres Ausgangsamin mehr vorhanden oder sein Gehalt auf den tolerierten Wert gesunken ist. Nach einer bevorzugten Arbeitsweise werden das tertiäre Amin und soviel Wasser vorgelegt, daß eine 15 bis 55gew%ige, vorzugsweise 20 bis 45gew.%ige, wäßrige Lösung vom tertiären Amin in Wasser vorliegt. Die Mischung wird auf 60 bis 98 °C erhitzt, vorzugsweise auf 70 bis 95 °C. Nun werden die ω-Monohalogencarbonsäure und das Alkalimetallhydroxid jeweils in Form der erwähnten wäßrigen Lösungen unter Aufrechterhaltung der genannten Temperatur im wesentlichen gleichzeitig dazugegeben (kontinuierlich oder portionsweise und getrennt voneinander), worauf die Mischung bei dieser Temperatur weiter gehalten wird, bis der angestrebte niedrige Amingehalt erreicht ist. Diese Reaktionszeit beträgt im allgemeinen 5 bis 30 Stunden. Was die Zugabe von Halogencarbonsäure und Alkalimetallhydroxid betrifft, hat es sich als vorteilhaft herausgestellt, zunächst die ω-Halogencarbonsäure allein zuzugeben und mit der Zugabe des Alkalimetallhydroxids erst dann zu beginnen, nachdem etwa 10 bis 40 Mol-%, vorzugsweise etwa 15 bis 30 Mol-%, von der ω-Halogencarbonsäure zugegeben sind. Nachdem also 10 bis 40 Mol-%, vorzugsweise 15 bis 30 Mol-%, von der insgesamt einzusetzenden Menge an ω-Monohalogencarbonsäure in die auf 60 bis 98 °C, vorzugsweise 70 bis 95 °C, erhitzte Amin/Wasser-Mischung kontinuierlich oder portionsweise eingebracht worden sind, werden das Alkalimetallhydroxid und die restliche Halogencarbonsäure bei der genannten Temperatur im wesentlichen gleichzeitig zugegeben (kontinuierlich oder portionsweise und getrennt voneinander). Nach der Zugabe von Alkalimetallhydroxid und Halogencarbonsäure wird die Mischung bei 60 bis 98 °C, vorzugsweise 70 bis 95 °C, zur Nachreaktion gehalten, im allgemeinen so lange, bis die genannten niedrigen Werte an tertiärem Ausgangsamin erreicht sind. Die so erhaltene wäßrige Betainlösung ist noch mehr oder weniger mit ω-Monohalogencarbonsäure verunreinigt.

Die mit ω-Monohalogencarbonsäure noch verunreinigte Betainlösung wird nun bei einem pH-Wert von 7,5 bis 13, vorzugsweise 8 bis 11, mit einem Sulfonierungsagens behandelt, um die anwesende ω-Monohalogencarbonsäure (sie liegt in Form eines Alkalimetallsalzes vor) in die entsprechende Sulfocarbonsäure (Sulfocarbonsäurealkalimetallsalz) umzuwandeln. Der genannte pH-Wert wird, sofern er nicht ohnehin bereits vorliegt, durch Zugabe von Alkalimetallhydroxid oder Säure (zum Beispiel Salzsäure) eingestellt. Die Mischung wird zur Sulfonierung auf eine Temperatur von 60 bis 98 °C gebracht, vorzugsweise auf 70 bis 95 °C. Diese Temperatur liegt in der Regel mit Abschluß des ersten Reaktionsschrittes (der Quaternisierung) bereits vor. Zur Sulfonierung können die üblichen Sulfonierungsmittel eingesetzt werden. Geeignete Sulfonierungsmittel sind also gasförmiges SO₂, H₂SO₃, Alkalimetallsulfit und Alkalimetallhydrogensulfit, wobei das Alkalimetall vorzugsweise Natrium oder Kalium ist. Von diesen Sulfonierungsmitteln sind die Sulfite, Pyrosulfite und Bisulfite (Hydrogensulfite) bevorzugt, die im allgemeinen in fester Form oder in Form einer 20 bis 40gew.%igen wäßrigen Lösung eingesetzt werden. Die Menge an Sulfonierungsagens, bezogen auf die anwesende Menge an ω-Monohalogencarbonsäure, beträgt in der Regel 1 bis 2,5 Moläquivalente, vorzugsweise 1,3 bis 2 Moläquivalente. Die Sulfonierung wird im einzelnen vorzugsweise derart durchgeführt, daß das Sulfonierungsagens in die auf 60 bis 98 °C, vorzugsweise 70 bis 95 °C, erhitzte wäßrige Betainlösung auf einmal oder kontinuierlich oder portionsweise eingebracht wird, worauf die Lösung so lange bei der genannten Temperatur gehalten wird, bis der angestrebte niedrige Gehalt an ω-Monohalogencarbonsäure erreicht ist. Diese Reaktionszeit beträgt im allgemeinen 1 bis 4 Stunden. Das so erhaltene Produkt stellt die bezüglich tertiärem Amin und Monohalogencarbonsäure reine wäßrige Betainlösung dar. Sofern das infolge eines eingesetzten Überschusses vorhandene Sulfonierungsagens unerwünscht ist, kann es zum Beispiel im Falle von Sulfit mit Sauerstoff (Luft) oder Wasserstoffperoxid zum Sulfat oxidiert und damit zerstört werden. Nach der oxidativen Behandlung liegt eine wäßrige Betainlösung vor, die auch frei ist vom eingesetzten Sulfonierungsmittel.

Bezüglich der Ausgangsverbindungen, tertiäres Amin, ω-Monohalogencarbonsäure und Alkalimetallhydroxid sei noch folgendes angemerkt: Die tertiären Ausgangsamine entsprechen der eingangs angegebenen Formel 2. Der lange Alkylrest R¹ kann auch Doppelbindungen enthalten, vorzugsweise 1 bis 3. Bevorzugte Ausgangsamine sind solche der Formel 2, wenn R¹ ein Alkylrest mit 8 bis 18 C-Atomen ist oder ein Rest der Formel R'CONH(CH₂)_{z}-, worin R' ein Alkylrest mit 5 bis 17 C-Atomen und z 2, 3 oder 4 ist, und R² und R³ jeweils Methyl sind. Als Beispiele seien genannt: Dimethyloctylamin, Dimethyllaurylamin, Dimethylstearylamin, Dimethylcocosalkylamin, Dimethyltalgalkylamin und dergleichen sowie Lauroylaminopropyldimethylamin, Stearoylaminopropyldimethylamin, Cocosacylaminopropyldimethylamin und dergleichen. Die ω-Halogencarbonsäure ist vorzugsweise die Monochloressigsäure. Das Alkalimetallhydroxid ist vorzugsweise Natriumhydroxid oder Kaliumhydroxid. Der Ausdruck "wäßrige" Betainlösung umfaßt auch solche Lösungen, die neben Wasser auch andere Lösungsmittel enthalten, zum Beispiel Methanol, Ethanol, Propanol und/oder Isopropanol.

Das erfindungsgemäße Verfahren weist eine Reihe von Vorteilen auf. Es liefert sehr reine wäßrige Betainlösungen. So können Betainlösungen erhalten werden, die weniger als 0,5 Gew.-% tertiäres Amin (bezogen auf die eingesetzte Menge an tertiärem Amin) und weniger als 50 ppm oder sogar weniger als 10 ppm ω-Monohalogencarbonsäure enthalten. Das erfindungsgemäße Verfahren ist ferner neben diskontinuierlich auch kontinuierlich durchführbar. Die kontinuierliche Arbeitsweise wird vorzugsweise in zwei bis vier kaskadenförmig angeordneten Rührkesseln durchgeführt.

Das erfindungsgemäße Verfahren kann also diskontinuierlich oder kontinuierlich durchgeführt werden und liefert das Betain in hoher Ausbeute und hoher Reinheit.

Die Erfindung wird nun an Beispielen noch näher erläutert.

### Beispiel 1

In einem 1-l-Glaskolben, ausgerüstet mit Rührer, Thermometer, Rückflußkühler und Tropftrichter, werden 188 g (0,587 mol) Cocosfettsäureamidopropyl-N,N-dimethylamin (Basis ist gehärtete Cocosfettsäure) und 345 g Wasser vorgelegt, das ist 35 Gew.-% tertiäre Aminverbindung in Wasser. Die Mischung wird unter Rühren auf 82 °C erwärmt. Zu dieser Suspension werden langsam und kontinuierlich in 5,5 Stunden 72,8 g (0,616 mol) einer 80gew.%igen wäßrigen Monochloressigsäure-(MCE)-Lösung getropft. Mit einer zeitlichen Verzögerung von 30 Minuten, nachdem etwa 1/5 (20 Mol-%) der gesamten MCE-Lösung zugegeben sind, werden 53,7 g (0,671 mol) einer 50gew.%igen wäßrigen NaOH-Lösung gleichzeitig mit der restlichen MCE-Lösung und getrennt davon kontinuierlich zugetropft (das Molverhältnis von tertiärer Aminverbindung, MCE und NaOH ist 1 zu 1,05 zu 1,14). Nach beendeter Zugabe wird 9 Stunden bei 80 °C nachreagieren gelassen. Die erhaltene 30gew.%ige wäßrige Betainlösung hat einen Gehalt an Ausgangsamidamin von 0,14 Gew.-% und an MCE von 0,13 Gew.-%, das sind 1300 ppm.

Zur erhaltenen Betainlösung, die einen pH-Wert von 10 bis 11 hat, werden bei einer Temperatur von 80 bis 85 °C unter Rühren 1,9 g (200 Mol-% oder 2 Moläquivalent, bezogen auf Rest-MCE) Natriumbisulfit als 30gew.%ige wäßrige Lösung gegeben, worauf man bei der Temperatur von 80 bis 85 °C und dem pH-Wert von 10 bis 11 nachreagieren läßt. Bereits nach 2 Stunden ist ein MCE-Gehalt von nur noch weniger als 5 ppm festzustellen.

Die so erhaltene, sowohl bezüglich Ausgangsamin als auch bezüglich MCE praktisch reine wäßrige Betainlösung wird zur Umwandlung des überschüssigen Natriumbisulfits in Natriumsulfat mit Salzsäure auf pH 5 eingestellt und 1 Stunde lang bei 85 °C mit 96 Mol-% Wasserstoffperoxid (Molprozente bezogen auf anwesendes Natriumbisulfit) gerührt. Es liegt die angestrebte reine und auch von Sulfit freie wäßrige 30gew.%ige Betainlösung vor.

### Beispiel 2

800 g einer analog Beispiel 1 hergestellten 30gew.%igen Cocosamidopropyl-N,N-dimethylcarboxymethylammoniumbetain-Lösung mit einem Ausgangsamidamin-Gehalt von nur noch 0,15 Gew.-% und einem MCE-Gehalt von 0,12 Gew.-% oder 1200 ppm und einem pH-Wert von 10 bis 11 werden mit 5,1 g (200 Mol-%, bezogen auf MCE) festem Natriumsulfit (Na₂SO₃ · 7H₂O) versetzt und 2 Stunden bei 90 bis 95 °C gerührt. Es liegt eine Natriumsulfit, aber nur noch weniger als 5 ppm MCE und 0,15 Gew.-% Ausgangsamidamin enthaltende wäßrige 30gew.%ige Betainlösung vor.

### Beispiel 3

In einem 1-l-Glaskolben, ausgerüstet mit Rührer, Thermometer, Rückflußkühler und Tropftrichter, werden 150 g (0,664 mol) Lauryldimethylamin (70 Gew.-% C₁₂, 25 Gew.-% C₁₄ und 5 Gew.-% C₁₆) und 300 g Wasser vorgelegt, das ist 33 Gew.-% tertiäre Aminverbindung in Wasser. Die Mischung wird unter Rühren auf 80 °C erwärmt. Zu dieser Suspension werden langsam und kontinuierlich in 5,5 Stunden 89,0 g (0,753 mol) einer 80gew.%igen wäßrigen Monochloressigsäure-(MCE)-Lösung getropft. Mit einer zeitlichen Verzögerung von 30 Minuten, nachdem etwa 1/5 (20 Mol-%) der gesamten MCE-Lösung zugegeben sind, werden 66,0 g (0,825 mol) einer 50gew.%igen wäßrigen NaOH-Lösung gleichzeitig mit der restlichen MCE-Lösung und getrennt davon kontinuierlich zugetropft (das Molverhältnis von tertiärem Amin, MCE und NaOH ist 1 zu 1,13 zu 1,24). Nach beendeter Zugabe wird 10 Stunden bei 80 °C nachreagieren gelassen. Die erhaltene 30gew.%ige wäßrige Betainlösung hat einen Gehalt an Ausgangsamin (Lauryldimethylamin) von 0,3 Gew.-% und an MCE von 0,2 Gew.-%, das sind 2000 ppm.

Zur erhaltenen Betainlösung, die einen pH-Wert von 10 bis 11 hat, werden bei einer Temperatur von 80 bis 85 °C unter Rühren 1,1 g (150 Mol-% oder 1,5 Moläquivalent, bezogen auf Rest-MCE) festes Natriumpyrosulfit gegeben, worauf man bei der Temperatur von 80 bis 85 °C und dem pH-Wert von 10 bis 11 nachreagieren läßt. Nach 4 Stunden ist ein MCE-Gehalt von nur noch weniger als 5 ppm festzustellen.

Die so erhaltene, sowohl bezüglich Ausgangsamin als auch bezüglich MCE praktisch reine wäßrige Betainlösung wird zur Umwandlung des überschüssigen Sulfits in Sulfat mit Salzsäure auf pH 5 eingestellt und 1,5 Stunden lang bei 85 °C mit 96 Mol-% Wasserstoffperoxid (Molprozente bezogen auf vorhandenes Sulfit) gerührt. Es liegt die angestrebte reine und auch von Sulfit freie wäßrige 30gew.%ige Betainlösung vor.

### Beispiele 4 und 5

Analog Beispiel 1 werden ausgehend von Octylamidopropyl-N,N-dimethylamin (Beispiel 4) und Laurylamidopropyl-N,N-dimethylamin (Beispiel 5) die entsprechenden Betainlösungen hergestellt. Durch Umsetzung mit Natriumbisulfit wie in Beispiel 1 wird die Menge an Rest-MCE auf weniger als 5 ppm gebracht.

### Beispiel 6

Analog Beispiel 3 wird ausgehend von Octyldimethylamin eine 30gew.%ige Betainlösung mit einem Rest-MCE-Gehalt von 2000 ppm und einem Restamingehalt von 0,3 Gew.-% hergestellt. Durch Umsetzung mit 2 Moläquivalenten Natriumsulfit, bezogen auf Rest-MCE, wie in Beispiel 3 beschrieben, wird ein Rest-MCE-Gehalt von weniger als 5 ppm erhalten.

## Patentansprüche

1. Verfahren zur Herstellung von reinen wäßrigen Lösungen von Betainen der allgemeinen Formel 1 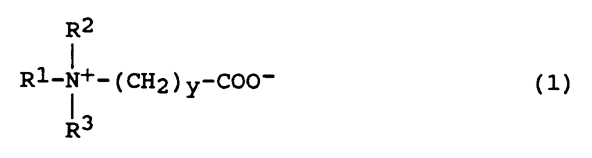 worin bedeuten
R¹ einen Alkylrest mit 6 bis 22 C-Atomen oder einen Rest der Formel R'CONH(CH₂)_{z}-, worin R' ein Alkylrest mit 5 bis 21 C-Atomen und z 2, 3 oder 4 ist,
R² einen Alkylrest mit 1 bis 4 C-Atomen oder einen Rest der Formel -(CH₂)ₘ-OH, worin m 1, 2 oder 3 ist,
R³ einen Alkylrest mit 1 bis 4 C-Atomen oder einen Rest der genannten Formel -(CH₂)ₘ-OH und
y 1, 2 oder 3,
durch Umsetzung von einem tertiären Amin der allgemeinen Formel 2
R¹-NR²R³ (2)
worin R¹, R² und R³ die angegebenen Bedeutungen haben,
mit einer ω-Halogencarbonsäure der allgemeinen Formel 3
X-(CH₂)_{y}-COOH (3)
worin X ein Halogen ist und y die genannte Bedeutung hat,
und mit Alkalimetallhydroxid in wäßriger Phase, dadurch gekennzeichnet, daß man zunächst das tertiäre Amin, die ω-Monohalogencarbonsäure und das Alkalimetallhydroxid im Molverhältnis von 1 zu 1 bis 1,5 zu 1 bis 1,8 bei einer Temperatur von 60 bis 98 °C umsetzt und dann die erhaltene wäßrige Betainlösung bei einem pH-Wert von 7,5 bis 13 und einer Temperatur von 60 bis 98 °C mit einem Sulfonierungsagens behandelt zur Überführung der in der wäßrigen Betainlösung vorhandenen ω-Monohalogencarbonsäure in die ω-Sulfocarbonsäure.

2. Verfahren nach Anspruch 1, dadurch gekennzeichnet, daß man das tertiäre Amin, die ω-Monohalogencarbonsäure und das Alkalimetallhydroxid im Molverhältnis von 1 zu 1,03 bis 1,3 zu 1 bis 1,5 einsetzt und bei einer Temperatur von 70 bis 95 °C umsetzt und dann die wäßrige Betainlösung bei einem pH-Wert von 8 bis 11 und einer Temperatur von 70 bis 95 °C mit einem Sulfonierungsagens behandelt.

3. Verfahren nach Anspruch 1 oder 2, dadurch gekennzeichnet, daß man die Umsetzung von tertiärem Amin, ω-Monohalogencarbonsäure und Alkalimetallhydroxid in der Weise durchführt, daß man das tertiäre Amin und soviel Wasser vorlegt, daß eine 15 bis 55gew.%ige Lösung von tertiärem Amin in Wasser vorliegt, diese Mischung auf eine Temperatur von 60 bis 98 °C erhitzt und in die erhitzte Mischung zunächst 10 bis 40 Mol-% von der einzusetzenden ω-Monohalogencarbonsäure und dann im wesentlichen gleichzeitig das Alkalimetallhydroxid und die restliche ω-Monohalogencarbonsäure einbringt, worauf die Mischung bei der genannten Temperatur zur Nachreaktion gehalten wird.

4. Verfahren nach Anspruch 1 oder 2, dadurch gekennzeichnet, daß man die Umsetzung von tertiärem Amin, ω-Monohalogencarbonsäure und Alkalimetallhydroxid in der Weise durchführt, daß man das tertiäre Amin und soviel Wasser vorlegt, daß eine 20 bis 45gew.%ige Lösung von tertiärem Amin in Wasser vorliegt, diese Mischung auf eine Temperatur von 70 bis 95 °C erhitzt und in die erhitzte Mischung zunächst 15 bis 30 Mol-% von der einzusetzenden ω-Monohalogencarbonsäure und dann im wesentlichen gleichzeitig das Alkalimetallhydroxid und die restliche ω-Monohalogencarbonsäure einbringt, worauf die Mischung bei der genannten Temperatur zur Nachreaktion gehalten wird.

5. Verfahren nach einem oder mehreren der Ansprüche 1 bis 4, dadurch gekennzeichnet, daß man das Sulfonierungsagens in einer Menge von 1 bis 2,5 Moläquivalenten einsetzt, bezogen auf vorhandene ω-Monohalogencarbonsäure.

6. Verfahren nach einem oder mehreren der Ansprüche 1 bis 4, dadurch gekennzeichnet, daß man das Sulfonierungsagens in einer Menge von 1,3 bis 2 Moläquivalenten einsetzt, bezogen auf vorhandene ω-Monohalogencarbonsäure.

7. Verfahren nach einem oder mehreren der Ansprüche 1 bis 6, dadurch gekennzeichnet, daß man als Sulfonierungsagens ein Alkalimetallsulfit, Alkalimetallpyrosulfit oder Alkalimetallbisulfit einsetzt.

8. Verfahren nach einem oder mehreren der Ansprüche 1 bis 7, dadurch gekennzeichnet, daß man die wäßrige Betainlösung nach der Sulfonierung mit Luft oder Wasserstoffperoxid behandelt zur Zerstörung des vorhandenen Sulfonierungsmittelrestes.

9. Verfahren nach einem oder mehreren der Ansprüche 1 bis 8, dadurch gekennzeichnet, daß als tertiäre Amine solche der Formel 2 eingesetzt werden, wenn R¹ ein Alkylrest mit 8 bis 18 C-Atomen ist oder ein Rest der Formel R'CONH(CH₂)_{z}-, worin R' ein Alkylrest mit 5 bis 17 C-Atomen und z 2, 3 oder 4 ist, und R² und R³ jeweils Methyl sind, als ω-Monohalogencarbonsäure die Monochloressigsäure und als Alkalimetallhydroxid Natriumhydroxid oder Kaliumhydroxid in Form einer 30 bis 60gew.%igen wäßrigen Lösung.

10. Verfahren nach einem oder mehreren der Ansprüche 1 bis 9, dadurch gekennzeichnet, daß insgesamt soviel Wasser eingesetzt wird, daß die fertige reine wäßrige Betainlösung 20 bis 60 Gew.-% Betain enthält, Gewichtsprozente bezogen auf die wäßrige Betainlösung.
